# EUROPÄISCHE PATENTSCHRIFT

(11) **EP 3 021 765 B1**
(45) Veröffentlichungstag und Bekanntmachung des Hinweises auf die Patenterteilung: **20.09.2017**
(21) Anmeldenummer: 14736763.5
(22) Anmeldetag: 04.07.2014
(51) Int. Cl.: A61B 17/14

(54) **WERKZEUG ZUM EINSETZEN IN EINE CHIRURGISCHEN SÄGE UND VERFAHREN ZUM FRÄSEN EINER NUT**
TOOL FOR MOUNTING IN A SURGICAL SAW AND METHOD OF MILLING A GROOVE
OUTIL A MONTER DANS UNE SCIE CHIRURGICALE ET PROCEDE DE FRAISER UNE RAINURE

(30) Priorität: 15.07.2013 DE 102013107485
(43) Veröffentlichungstag der Anmeldung: 25.05.2016
(73) Patentinhaber: Reng, Wolfgang, 82467 Garmisch-Partenkirchen (DE)
(72) Erfinder: Reng, Wolfgang, 82467 Garmisch-Partenkirchen (DE)
(74) Vertreter: Twelmeier Mommer & Partner
(86) Internationale Anmeldenummer: PCT/EP2014/064312
(87) Internationale Veröffentlichungsnummer: WO 2015/007546

(56) Entgegenhaltungen:
- EP-A2- 1 974 679
- WO-A1-93/01751
- US-A- 67 369
- US-A1- 2009 093 814
- US-A1- 2011 092 975
- US-A1- 2012 130 380

## Beschreibung

Die Erfindung betrifft ein Werkzeug zum Einsetzen in eine chirurgische Stichsäge mit den Merkmalen des Oberbegriffs des Anspruchs 1. Ein Werkzeug zum Einsetzen in eine chirurgische Stichsäge mit einem an eine Werkzeugaufnahme der chirurgischen Stichsäge angepassten Aufnahmebereich und mit einem Kopf, der von der chirurgischen Stichsäge entlang einer Arbeitsrichtung linear oszillierend bewegbar ist und mehrere Zähne aufweist, die in Arbeitsrichtung hintereinander angeordnet sind, ist aus der US D622,383 S, der US D622,386 S sowie der US 2011/0092975 A1 bekannt. Das bekannte Werkzeug weist an seinem Kopf zwei parallele Sägeblätter mit mehreren zueinander verschränkten Zähnen auf. Die beiden Sägeblätter sind in einem Abstand zueinander angeordnet, sodass sich mit dem Werkzeug zwei parallele Schlitze sägen lassen.

Das Werkzeug ist ein Operationsinstrument in der Humanmedizin. Es kann bei der Präparation des knöchernen Lagers bei der Implantation eines künstlichen Gelenkersatzes, insbesondere bei Kniegelenken, eingesetzt werden. Endoprothesen werden oftmals in Nuten befestigt, die in den Knochen eingebracht werden. Anschließend wird das Implantat in die Nut einzementiert oder zementfrei in die Nut eingeschlagen. Insbesondere bei der zementfreien Befestigung muss die Nut im Knochen sehr präzise präpariert werden.

Um die Präzision bei der Präparation der Nut in dem Knochen zu steigern, ist es ferner bekannt, eine Schablone auf dem Knochen zu befestigen, welche einen Schlitz aufweist, der in seiner Länge und Breite der Länge und Breite der in dem darunter liegenden Knochen auszusägenden Nut entspricht. Das eingangs erwähnte, bekannte Werkzeug mit den beiden parallelen Sägeblättern an seinem Kopf hat dort eine Breite, die der Breite der auszusägenden Nut entspricht. Die Länge der Sägeblätter ist kürzer als die auszusägende Nut. Das bekannte Werkzeug wird in eine chirurgische Stichsäge eingesetzt, von dieser in eine lineare oszillierende Bewegung versetzt und mit seinen am Kopf angeordneten Sägeblättern in den Schlitz der auf dem Knochen befestigten Schablone eingeführt. Der Kopf des Werkzeugs mit den Sägeblättern steht über einer am Werkzeug vorgesehenen Führungsfläche vor, sodass die Führungsfläche an der Oberfläche der Schablone aufliegt, wenn die Sägeblätter eine bestimmte Tiefe im Knochen erreicht haben. Mit dem bekannten Werkzeug lassen sich somit zwei parallele Schlitze in den Knochen sägen, deren Abstand der gewünschten Nutbreite, deren Länge der gewünschten Nutlänge und deren Tiefe der gewünschten Nuttiefe entspricht. Zwischen den beiden Schlitzen bleibt ein in Längsrichtung der Nut verlaufender Knochensteg stehen. Bei dem bekannten Verfahren wird anschließend dieser Knochensteg mit einem handgeführten, tiefenbegrenzten Spezialmeißel entfernt. Der Spezialmeißel muss manuell ziehend und schiebend durch die Nut geführt werden, wobei ein seitliches Kippen des Spezialmeißels auftreten kann. Die freihändige Präparation der Nut mit dem Spezialmeißel kann folglich zu einer erhöhten Varianz der Knochennutabmessungen führen. Insbesondere bei Strukturveränderungen des Knochens im Bereich der Nut (Knochensklerose, Knochennarben, Fremdkörper, Zysten oder Osteoporose) kann ein deutlich veränderter Kraftaufwand bei der Führung des Spezialmeißels entstehen, durch den die Präparationsgenauigkeit verringert wird. Das bekannte Werkzeug ist zudem nur für eine einmalige Verwendung zugelassen, sodass für jede Operation ein neues Sägewerkzeug notwendig ist und dadurch hohe Operationskosten entstehen.

Aus der WO 93/01751 A1 und der EP 1 974 679 A2 sind ferner Werkzeuge zum Einsetzen in chirurgische Sägen anderer Art bekannt, welche das Werkzeug jeweils in eine oszillierende Schwenkbewegung versetzen. Diese gattungsfremden Werkzeuge weisen jeweils einen Kopf auf, welcher von der chirurgischen Säge auf einer Kreisbahn verschwenkt wird und zwei Sätze von Zähnen mit entgegengesetzter Arbeitsrichtung aufweist. Jeder der Zähne enthält eine vordere Fläche zum Schneiden von Knochenmaterial und rückseitige Flächen zum Herausfördern von Spänen, wobei die vorderseitige Fläche in Bezug auf die Arbeitsrichtung stärker als die rückseitigen Flächen geneigt ist. Die US 67,369 A offenbart eine Säge, welche zwischen zwei Schneidezähnen einen Reinigungszahn aufweist, welcher leicht konkave Abschnitte zum Reinigen des Schnittspalts aufweist.

Der vorliegenden Erfindung liegt die Aufgabe zugrunde, ein Werkzeug zum Einsetzen in eine chirurgische Säge zu schaffen, welches verbesserte Eigenschaften aufweist.

Die Aufgabe wird durch ein Werkzeug mit den Merkmalen des Anspruchs 1 gelöst. Das erfindungsgemäße Werkzeug hat einen an eine Werkzeugaufnahme einer chirurgischen Säge angepassten Aufnahmebereich und einen Kopf, der von der chirurgischen Säge entlang einer Arbeitsrichtung oszillierend bewegbar ist. Das Werkzeug ist für chirurgische Stichsägen geeignet, die eine lineare oszillierende Bewegung ausführen. Am Kopf des Werkzeugs sind mehrere Zähne vorgesehen. Zumindest zwei der Zähne sind in Arbeitsrichtung hintereinander angeordnet und können von der Säge entlang der Arbeitsrichtung oszillierend vor- und zurückbewegt werden. Der Kopf des Werkzeugs weist einen ersten und einen zweiten Zahn zum Abfräsen von Knochenmaterial auf. Der Kopf kann hierzu dem Knochen in einer quer zur Arbeitsrichtung orientierten Zustellbewegung zugestellt werden. Jeder der Zähne enthält eine erste Fläche zum Abschaben von Knochenmaterial, die quer zur Richtung der oszillierenden Bewegung orientiert ist und im Folgenden als "Schabefläche" bezeichnet wird. Jeder der Zähne enthält eine zweite Fläche zum Andrücken von Knochenmaterial, die an die Schabefläche angrenzt und im Folgenden als "Andrückfläche" bezeichnet wird. Die Schabefläche ist in Bezug auf die Arbeitsrichtung stärker als die Andrückfläche geneigt. Die beiden Zähne sind mit ihrer Schabefläche und ihrer Andrückfläche entgegengesetzt orientiert, sodass bei einer Bewegung des Kopfes in Arbeitsrichtung jeweils die Schabefläche des in Arbeitsrichtung vorne liegenden Zahnes und die Andrückfläche des in Arbeitsrichtung dahinter liegenden Zahnes arbeiten.

Die Erfindung betrifft ferner ein Verfahren zum Fräsen einer Nut in einen Knochen mit einem in eine chirurgische Stichsäge eingesetzten Werkzeug, welches einen Kopf mit mehreren Zähnen aufweist, der von der Stichsäge entlang einer Arbeitsrichtung linear oszillierend vor- und zurückbewegt wird. Bei der Vorwärtsbewegung des Kopfes schabt eine in Arbeitsrichtung vorne liegende Schabefläche eines ersten Zahnes Knochenmaterial ab und eine in Arbeitsrichtung hinter der Schabefläche des ersten Zahnes liegende Andrückfläche eines zweiten Zahnes drückt Knochenmaterial an den Knochen an. Bei der Rückwärtsbewegung des Kopfes schabt eine in Arbeitsrichtung vorne liegende Schabefläche des zweiten Zahnes Knochenmaterial ab und eine in Arbeitsrichtung hinter der Schabefläche des zweiten Zahnes liegende Andrückfläche des ersten Zahnes drückt Knochenmaterial an den Knochen an. Das erfindungsgemäße Verfahren wird an einem toten menschlichen oder tierischen Körper oder an einem Knochen außerhalb des lebenden menschlichen oder tierischen Körpers durchgeführt. Es kann beispielsweise zu Zwecken der Ausbildung von Chirurgen, der Erprobung eines erfindungsgemäßen Werkzeugs und/oder der Erprobung eines Implantates durchgeführt werden.

Die Erfindung hat wesentliche Vorteile:
- Das erfindungsgemäße Werkzeug kann wie ein Sägeblatt an einer maschinellen chirurgischen Stichsäge betrieben werden. Bauliche Änderungen an der chirurgischen Säge sind nicht erforderlich.
- Im Gegensatz zu den bekannten Sägeblättern wird das Knochenmaterial nicht zerschnitten. Das erfindungsgemäße Werkzeug weist deshalb keine zueinander verschränkten Sägezähne auf. Das Knochenmaterial wird durch die erfindungsgemäße Ausgestaltung nicht geschnitten sondern vielmehr abgeschabt oder abgefräst. Dadurch lässt sich eine sehr gute und saubere Oberfläche des Knochens erreichen. Die von der Schabefläche bei der Vorwärtsbewegung in Arbeitsrichtung ausgeführte Schabefunktion ist somit eher mit einem Knochenhobel vergleichbar.
- Mit dem erfindungsgemäßen Werkzeug sollen die abgeschabten Knochenpartikel nicht wie bei herkömmlichen Sägeblättern aus der Nut heraus befördert werden. Vielmehr soll das abgeschabte Knochenmaterial durch die Andrückflächen an den Knochen im Bereich der Nutränder angedrückt werden. Durch das Einpressen von Knochenpartikeln in die Nutenränder wird das Knochenmaterial im Bereich der Nutränder verdichtet. Durch die Verdichtung lässt sich eine Erhöhung der Festigkeit des Knochenmaterials erreichen, die zu einer verbesserten Haltbarkeit und erhöhten Belastbarkeit des in die Nut eingesetzten Implantats führt.

- Dadurch, dass die beiden Zähne mit ihrer Schabefläche und ihrer Andrückfläche entgegengesetzt orientiert sind, ist gewährleistet, dass sowohl in der Vorwärtsbewegung des Kopfes als auch in der Rückwärtsbewegung des Kopfes jeweils eine Schabefläche und eine in Arbeitsrichtung dahinter liegende Andrückfläche arbeitet. Die Andrückfläche ist in Bezug auf die Arbeitsrichtung relativ gering geneigt, um ein gutes Andrücken oder "Anstreichen" des Knochenmaterials an die knöcherne Kontaktfläche zu gewährleisten.
- Mit dem erfindungsgemäßen Werkzeug kann die benötigte Nut in dem Knochen in einem Arbeitsschritt gefräst werden. Die unpräzise Endpräparation mit dem Spezialmeißel ist nicht mehr nötig. Hierdurch wird eine sehr hohe Genauigkeit bei der Nutpräparation erreicht und die gesamte Operationszeit verkürzt. Die Varianz der Knochennutdimensionen kann sowohl im homogen-spongiösen als auch im inhomogenen Knochen verringert werden. Dadurch kann in stärkerem Umfang als bisher ein zementfreies Einsetzen des Implantates durchgeführt werden.
- Durch das erfindungsgemäße Werkzeug lässt sich die allgemeine und die spezielle Komplikationsrate verringern. Dies bedeutet insbesondere eine verringerte Rate an Wundinfektionen, verringertem postoperativem Schmerzmittel- und Infusionsbedarf und einer verkürzten Rekonvaleszenz dauer. Es lassen sich die direkten Operationskosten und die allgemeinen Krankenhauskosten verringern.

Das erfindungsgemäße Werkzeug ist insbesondere sehr gut für die Implantation der tibialen Anteile eines Kniegelenk-Implantates der Firma Biomet, Warsaw, Indiana, USA geeignet, das als "Oxford Unikondyläres Kniesystem" bekannt ist und bei welchem bisher das eingangs beschriebene Werkzeug mit den beiden parallelen Sägeblättern eingesetzt wird. Die Vorteile der Erfindung treten hierbei in besonderem Maße zu Tage. Der tibiale Anteil der Oxford Unischlitten-Endoprothese verfügt über eine perforierte Finne zur Verankerung im tibialen spongiösen Knochenlager. Die Verankerungsfinne ist quer zur Längsrichtung der Nut gesehen am vorderen und hinteren Ende abgerundet. Mit dem erfindungsgemäßen Werkzeug lässt sich eine Nut fräsen, die genau an diese Rundungen der Finne angepasst ist. Die bei dem bekannten Verfahren am vorderen und hinteren Ende der Nut durch den handgeführten Spezialmeißel tiefer als von der Kontur der Verankerungsfinne benötigte Ausschabung der Nut entfällt. Der Sitz der Verankerungsfinne in der Nut wird dadurch verbessert.

Das erfindungsgemäße Werkzeug ist bevorzugt für eine chirurgische Mehrfachverwendung zugelassen. Hierfür kann das Werkzeug einstückig ausgeführt sein. Es ist vorteilhafterweise hochglanzpoliert. Das Werkzeug ist hinterschnittfrei ausgeführt und weist keine Löcher, Hohlräume oder andere Konturen auf, in denen sich Knochen- und Gewebereste festsetzen. Hierdurch wird eine maschinelle Reinigung im Rahmen der Sterilgutaufbereitung ermöglicht. Außerdem sind alle Oberflächen für die Qualitätsprüfung bei der Sterilisation einsehbar. Das Werkzeug besteht bevorzugt aus einem nichtrostenden Chromstahl ("Chirurgenstahl"). Bevorzugt ist ein martensitischer Chromstahl mit einem Chrom-Anteil von mindestens 18 %, wie er beispielsweise unter der genormten Werkstoffnummer 1.4112 erhältlich ist. Für eine Zulassung zur Mehrfachverwendung erfüllt das Werkzeug die einschlägigen Vorschriften, insbesondere die Richtlinie 93/42/EWG, das Medizinproduktegesetz, die Medizinprodukte-Betreiberverordnung und die vom Robert Koch Institut aufgestellten Richtlinien zur Wiederverwendung.

In vorteilhafter Ausgestaltung der Erfindung enthält jeder der Zähne eine am Übergang von der Schabefläche zur Andrückfläche gebildete Kante, die im Folgenden als "Hauptschneide" bezeichnet wird. Die Hauptschneide des ersten Zahnes kann parallel zur Hauptschneide des zweiten Zahnes verlaufen. Die Hauptschneide kann einen Winkel von mindestens 75° zur Arbeitsrichtung aufweisen, insbesondere kann sie senkrecht zur Arbeitsrichtung verlaufen. Die Hauptschneide verläuft bevorzugt gradlinig. Der Abstand der Hauptscheide des ersten Zahnes zu der Hauptschneide des zweiten Zahnes - parallel zur Arbeitsrichtung gemessen - ist insbesondere höchstens so groß wie der Arbeitshub der Säge, für die das Werkzeug bestimmt ist. Der Abstand der Hauptschneide des ersten Zahnes zu der Hauptschneide des zweiten Zahnes kann 2 mm bis 10 mm, insbesondere 3 mm bis 8 mm, betragen. Hierdurch lässt sich eine besonders geeignete Zahnform erreichen, bei der die Zähne nicht zueinander verschränkt sind. Die mit dem Werkzeug geschaffene Oberfläche in der Knochennut lässt sich dadurch verbessern.

Das Werkzeug kann eine Führungsfläche zum Begrenzen der Frästiefe des Werkzeugs in dem Knochenmaterial aufweisen, wobei der Kopf des Werkzeugs über der Führungsfläche vorsteht und die Führungsfläche parallel zur Arbeitsrichtung orientiert ist. Die Führungsfläche kann sich am Werkzeug in Arbeitsrichtung gesehen auf beiden Seiten des Kopfes erstrecken. Die Höhe der Hauptschneiden über der Führungsfläche richtet sich nach der gewünschten Nuttiefe und kann 8 mm bis 20 mm, insbesondere 10 mm bis 15 mm, betragen. Vor dem Fräsen der Nut in dem Knochen kann eine einen Schlitz enthaltende Frässchablone auf den Knochen aufgesetzt und dort fixiert werden. Anschließend kann der Kopf des Werkzeugs mit einer quer zur Arbeitsrichtung orientierten Zustellbewegung in den Schlitz der Frässchablone eingeführt werden, um eine Nut in das unter der Frässchablone liegende Knochenmaterial zu fräsen, die in ihrer Länge der Länge des Schlitzes in der Frässchablone und in ihrer Breite der Breite des Kopfes des Werkzeugs entspricht. Die Führungsfläche dient zum Begrenzen der Zustellbewegung des Kopfes in Tiefenrichtung der zu fräsenden Nut, insbesondere zum Anlegen des Werkzeugs an die auf den Knochen aufgesetzte Frässchablone. Bevorzugt entspricht die Breite einer der Hauptschneiden quer zur Arbeitsrichtung der Breite der zu fräsenden Nut. Hierdurch kann die Nut in ihrer vollen Breite durch das erfindungsgemäße Werkzeug in nur einem Arbeitsschritt präpariert werden, sodass kein zusätzlicher Arbeitsschritt mit einem Spezialmeißel mehr erforderlich ist.

Wird nach dem Fräsen der Nut ein Implantat in die Nut eingesetzt, so kann die Nutbreite in Abhängigkeit von der Breite des in die Nut einzusetzenden Teils des Implantats und in Abhängigkeit von der Art der Verankerung des Implantats gefräst werden. Bei einer Verankerung, bei der das Implantat einzementiert wird, wird die Nut breiter als die Breite des in die Nut einzusetzenden Teils des Implantats gefräst. Bei einer "fit and fill"-Verankerung, welche für eine zementierte und zementfreie Verankerung geeignet ist, entspricht die Nutbreite der Breite des in die Nut einzusetzenden Teils des Implantats. Bei einer "pressfit"-Verankerung, bei der das Implantat mit einer Klemmpassung ohne Zement eingesetzt wird, wird die Nut schmaler als die Breite des in die Nut einzusetzenden Teils des Implantats gefräst. Bei einer "pressfit"-Verankerung kann die Nut 0,1 mm bis 0,4 mm, insbesondere 0,3 mm, schmaler als die Breite des in die Nut einzusetzenden Teils des Implantats, zum Beispiel der Verankerungsfinne, gefräst werden. Insbesondere bei der zementfreien Befestigung darf die Nut weder zu breit - dann würde die Verankerungsfinne nicht fest sitzen - noch zu schmal - dann könnte der Knochen, insbesondere wenn er sklerotisch verändert ist, beim Einpressen der Verankerungsfinne Risse bekommen - sein. Durch die genannten Abmessungen lässt sich eine gut geeignete Presspassung zwischen Nut und Verankerungsfinne erreichen, die eine zuverlässige zementfreie Befestigung des Implantats in der Nut ermöglicht.

In weiterer vorteilhafter Ausgestaltung der Erfindung verändert die Andrückfläche in einem an die Hauptschneide angrenzenden Abschnitt ihren Abstand zu einer Referenzlinie höchstens um 0,2 mm, insbesondere um höchstens 0,17 mm, wenn der Abstand der Andrückfläche zu der Referenzlinie an zwei Stellen gemessen wird, die entlang der Referenzlinie 0,2 mm voneinander beanstandet sind. Die Referenzlinie ist eine Gerade, die parallel zu der Arbeitsrichtung an die Hauptschneide des ersten Zahnes und/oder die Hauptschneide des zweiten Zahnes angelegt wird. Die Referenzlinie kann so an die Hauptschneide des ersten Zahnes und die Hauptschneide des zweiten Zahnes angelegt werden, dass während der oszillierenden Bewegung des Kopfes in Arbeitsrichtung eine definierte Stelle auf dem Knochen sowohl von dem Berührpunkt der Referenzlinie mit der Hauptschneide des ersten Zahnes als auch von dem Berührpunkt der Referenzlinie mit der Hauptschneide des zweiten Zahnes überstrichen wird. Die Referenzlinie kann in einer Referenzebene liegen, welche die Hauptschneide des ersten Zahnes und die Hauptschneide des zweiten Zahnes schneidet. Die Referenzebene ist eine parallel zur Arbeitsrichtung liegende Ebene, die zugleich parallel zu einer Zustellbewegung liegt, mit welcher die Zähne quer zur Arbeitsrichtung dem Knochen beim Fräsen der Nut zustellbar sind. Die Referenzebene kann senkrecht zur Führungsfläche liegen. Die Referenzlinie kann eine Gerade sein, die in der Referenzebene an die Hauptschneide des ersten Zahnes und die Hauptschneide des zweiten Zahnes angelegt wird. Der an die Hauptschneide angrenzende Abschnitt der Andrückfläche erstreckt sich entlang der Referenzlinie bis zu einem Abstand von 0,5 mm, insbesondere 1 mm, von der Hauptschneide. Die Schabefläche kann in einem an die Hauptschneide angrenzenden Abschnitt ihren Abstand zu der Referenzlinie mindestens um 0,18 mm, insbesondere um mindestens 0,19 mm, verändern, wenn der Abstand der Schabefläche zu der Referenzlinie an zwei Stellen gemessen wird, die entlang der Schabefläche 0,2 mm voneinander beanstandet sind. Der an die Hauptschneide angrenzende Abschnitt der Schabefläche erstreckt sich entlang der Schabefläche bis zu einem Abstand von 0,5 mm, insbesondere 1 mm, von der Hauptschneide. Der Abstand der Andrückfläche zu der Referenzlinie und der Abstand der Schabefläche zu der Referenzlinie werden senkrecht zu der Referenzlinie gemessen. Schabefläche und/oder Andrückfläche können gradlinig oder gekrümmt verlaufen.

Durch die an zwei voneinander beabstandeten Stellen durchgeführte Differenzmessung des Abstandes zu der Referenzlinie wird ein Wert für die Neigung der Schabefläche beziehungsweise der Andrückfläche gegenüber der Arbeitsrichtung definiert, der unabhängig von einer Krümmung der Andrückfläche oder der Schabefläche ist. Die Neigung der Schabefläche beziehungsweise der Andrückfläche bestimmt, wie stark die Schabefläche beziehungsweise Andrückfläche bei einer Verschiebung des Kopfes um einen vorbestimmten Weg in Arbeitsrichtung ihren Abstand von der Referenzlinie verändert, und wie stark die Schabe- beziehungsweise Andrückwirkung der jeweiligen Fläche ist. Die Zahnkontur zwischen der Hauptschneide des ersten Zahnes und der Hauptschneide des zweiten Zahnes kann einen Abstand zu der Referenzlinie aufweisen, der höchstens 25 %, insbesondere 10 % bis 20 %, des entlang der Referenzlinie gemessenen Abstandes von der Hauptschneide des ersten Zahnes zu der Hauptschneide des zweiten Zahnes beträgt. Es hat sich überraschend gezeigt, dass sich mit einem die genannten Abmessungen aufweisenden Werkzeug eine besonders gute Oberflächengüte der Nutoberfläche erreichen lässt.

In weiterer Ausgestaltung der Erfindung kann die Schabefläche eine ebene Fläche sein, welche einen Winkel von mindestens 75°, insbesondere von mindestens 85°, zur Arbeitsrichtung aufweist. Die ebene Schabefläche kann senkrecht zur Arbeitsrichtung orientiert sein, also den größtmöglichen Winkel von 90° zur Arbeitsrichtung aufweisen. Der Winkel zwischen der ebenen Schabefläche und der Arbeitsrichtung kann in der Referenzebene gemessen werden, während gleichzeitig die ebene Schabefläche senkrecht zur Referenzebene verläuft.

In bevorzugter Ausgestaltung kann die Andrückfläche des ersten Zahnes und die Andrückfläche des zweiten Zahnes konkav gewölbt sein. Die Andrückfläche kann in einer Schnittansicht, bei der die Schnittebene parallel zur Referenzebene durch die Hauptschneide verläuft, einen Krümmungsradius von mindestens 2 mm, insbesondere von 3 mm bis 10 mm, aufweisen. Bevorzugt beträgt der Krümmungsradius 4 mm bis 6 mm. Hierdurch lässt sich ein besonders gutes Andrücken von abgeschabtem Knochenmaterial an den Knochen erreichen. Es kann vorteilhaft sein, wenn die Andrückfläche des ersten Zahnes und die Andrückfläche des zweiten Zahnes von der Oberfläche eines Kreiszylinders gebildet werden. Dabei kann die Andrückfläche des ersten Zahnes knickfrei in die Andrückfläche des zweiten Zahnes übergehen. Die Mittellinie des Kreiszylinders kann senkrecht zur Arbeitsrichtung orientiert sein. Sie kann senkrecht zur Referenzebene, und insbesondere auch parallel zur Führungsfläche, liegen. Die Andrückflächen können senkrecht zur Referenzebene verlaufen. Der Kreiszylinder hat vorteilhafterweise einen Durchmesser von mindestens 5 mm, insbesondere von 7 mm bis 12 mm.

Es kann vorteilhaft sein, wenn der Kopf des Werkzeugs Seitenflächen aufweist, die parallel zur Arbeitsrichtung orientiert sind. Bevorzugt hat der Kopf ebene Seitenflächen. Die Seitenflächen sind vorteilhafterweise senkrecht zu der Führungsfläche. In einer Schnittansicht, bei der die Schnittebene senkrecht zur Arbeitsrichtung durch den Kopf verläuft, kann jeder Zahn einen rechteckigen Querschnitt aufweisen. Durch eine solche Gestaltung der Seitenflächen des Kopfes lässt sich auch an den Seitenfläche der Nut eine gute Verdichtung des Knochenmaterials erreichen.

Das Werkzeug enthält einen Körper mit einer Längsrichtung, wobei an einem ersten Ende des Körpers der Kopf des Werkzeugs angeordnet ist und wobei an einem zweiten Ende des Körpers der an die Werkzeugaufnahme der chirurgischen Stichsäge angepasste Aufnahmebereich angeordnet ist, über den das Werkzeug von der chirurgischen Stichsäge in einer parallel zur Längsrichtung des Körpers orientierten Arbeitsrichtung linear bewegbar ist. Der Aufnahmebereich des Werkzeugs kann einen parallel zur Arbeitsrichtung verlaufenden Oberflächenabschnitt aufweisen.

Weitere Vorteile und Merkmale der Erfindung ergeben sich aus der nachfolgenden Beschreibung eines Ausführungsbeispiels in Zusammenhang mit den Figuren.

Es zeigen:
- Figur 1: eine Schrägansicht eines erfindungsgemäßen Werkzeugs,
- Figur 2: eine schematische Schrägansicht auf den Kopf eines erfindungsgemäßen Werkzeugs,
- Figur 3: eine stark vergrößerte Ansicht in Richtung des Pfeiles III der Figur 2 auf den ersten und den zweiten Zahn des Kopfes.

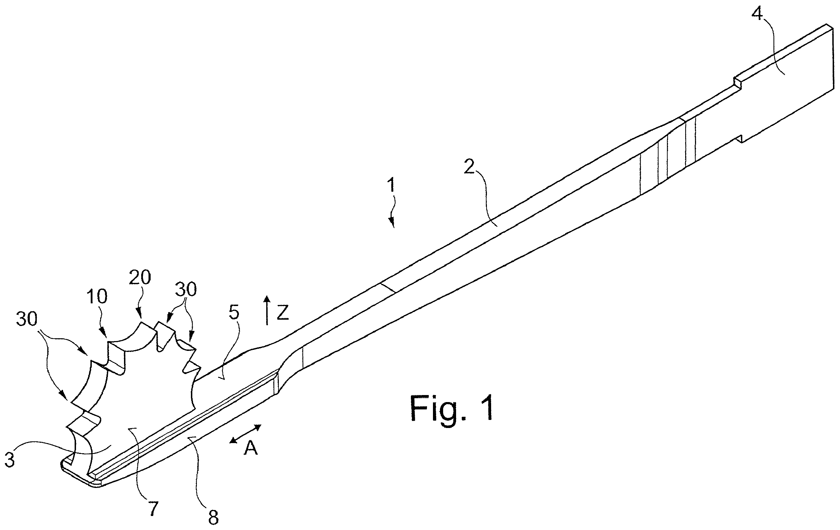

In Figur 1 ist ein Werkzeug 1 zum Einsetzen in eine chirurgische Stichsäge dargestellt. Das Werkzeug 1 hat einen länglichen Körper 2 mit einer Längsrichtung. An einem ersten Ende des Körpers 2 ist ein Kopf 3 angeordnet. An einem zweiten Ende des Körpers 2 ist ein Aufnahmebereich 4 angeordnet der an eine Werkzeugaufnahme der chirurgischen Stichsäge angepasst ist. Das Werkzeug 1 wird mit dem Aufnahmebereich 4 in die Werkzeugaufnahme der chirurgischen Stichsäge eingesetzt und wird, wenn die chirurgische Stichsäge eingeschaltet wird, von der chirurgischen Stichsäge parallel zur Längsrichtung des Körpers 2 vor- und zurückbewegt. Die Richtung der oszillierenden Bewegung des Werkzeugs 1 ist in Figur 1 durch den mit dem Buchstaben A gekennzeichneten Doppelpfeil angedeutet und wird als Arbeitsrichtung bezeichnet. Der Kopf 3 weist einen ersten Zahn 10 und einen zweiten Zahn 20 zum Abfräsen von Knochenmaterial auf. Die Ausgestaltung der Zähne 10 und 20 wird im Folgenden noch näher erläutert werden. Neben den Zähnen 10 und 20 kann der Kopf 3 weitere Zähne 30 aufweisen, die ähnlich wie die Zähne 10 und 20 gestaltet sind. An dem Körper 2 ist eine Führungsfläche 5 zum Begrenzen der Frästiefe des Werkzeugs 1 in dem Knochenmaterial vorgesehen. Der Kopf 3 steht über der Führungsfläche 5 vor. Die Führungsfläche 5 umgibt den Kopf 3 auf drei Seiten. Die Führungsfläche 5 ist parallel zur Arbeitsrichtung A.

Das in Figur 1 dargestellte Werkzeug 1 kann in einem Verfahren zum Fräsen einer Nut in einen beliebigen Knochen verwendet werden. Es ist besonders gut zum Fräsen einer Nut in einen Tibiaknochen geeignet und wird deshalb im Folgenden beispielhaft an dieser Anwendung beschrieben. In vorbereitenden Verfahrensschritten wird ein Plateau an der Tibia präpariert. Auf dem Plateau wird eine einen Schlitz enthaltende Frässchablone aufgesetzt und dort fixiert. Das in die Stichsäge eingesetzte Werkzeug 1 wird bei laufender Stichsäge mit dem Kopf 3 in den Schlitz der Frässchablone eingeführt, dann wird der Kopf 3 dem Knochen mit einer quer zur Arbeitsrichtung A orientierten Zustellbewegung Z zugestellt. Der Kopf 3 mit den Zähnen 10, 20, 30 fräst Knochenmaterial ab, sodass der Kopf 3 in den Knochen eintaucht und eine Nut in dem Knochen entsteht. Der Kopf 3 wird so weit in Richtung Z bewegt, bis die Führungsfläche 5 an der Frässchablone anliegt. Die Breite des Kopfes 3 entspricht der Breite des Schlitzes in der Frässchablone und der Breite der in den Knochen zu fräsenden Nut. Die chirurgische Stichsäge mit dem Werkzeug 1 wird außerdem in Arbeitsrichtung A vor- und zurückgeschoben, bis der Kopf 3 den Anfang und das Ende des Schlitzes in der Frässchablone erreicht hat. Hierdurch wird gewährleistet, dass die in den Knochen gefräste Nut in ihrer Länge der Länge des Schlitzes in der Frässchablone entspricht.

Das Werkzeug 1 und die Frässchablone sind in ihren Abmessungen auf das einzusetzende Tibiaimplantat abgestimmt. Der Abstand der Seitenflächen 7 des Kopfes 3 definiert seine Breite, welche der Breite der zu fräsenden Nut entspricht. Die Breite des Schlitzes in der Frässchablone kann der Breite des Kopfes 3 entsprechen, sodass das Werkzeug 1 über die Seitenflächen 7 in der Frässchablone zentriert und geführt ist. Wenn die Breite des Kopfes 3 geringer als die Breite des Schlitzes in der Frässchablone ist, kann der Körper 2 zusätzliche ebene Zentrierflächen 8 enthalten, die parallel zur Arbeitsrichtung A und senkrecht zur Führungsfläche 5 orientiert sind und in einer entsprechenden Nut in der Frässchablone geführt werden können.

Nach dem Fräsen der Nut wird der Kopf 3 des Werkzeugs 1 aus der gefrästen Nut heraus geführt und die Frässchablone von der Tibia abgenommen. Das Tibiaimplantat kann ein Teil eines künstlichen Kniegelenks sein und eine Verankerungsfinne aufweisen, welche dann in die gefräste Nut eingesetzt wird. Die Präparation der Nut in der Tibia ist damit nach nur einem Arbeitsschritt mit dem Werkzeug 1 abgeschlossen. Soll das Tibiaimplantat zementfrei eingesetzt werden, so wird die Nut 0,3 mm schmaler als die Verankerungsfinne des Tibiaimplantats gefräst. Durch den Überschnitt von 0,3 mm wird eine "pressfit"-Verankerung erreicht, das heißt, das Tibiaimplantat kann in die Tibia eingeschlagen werden und wird dort ohne weitere Verwendung von Zement sicher gehalten.

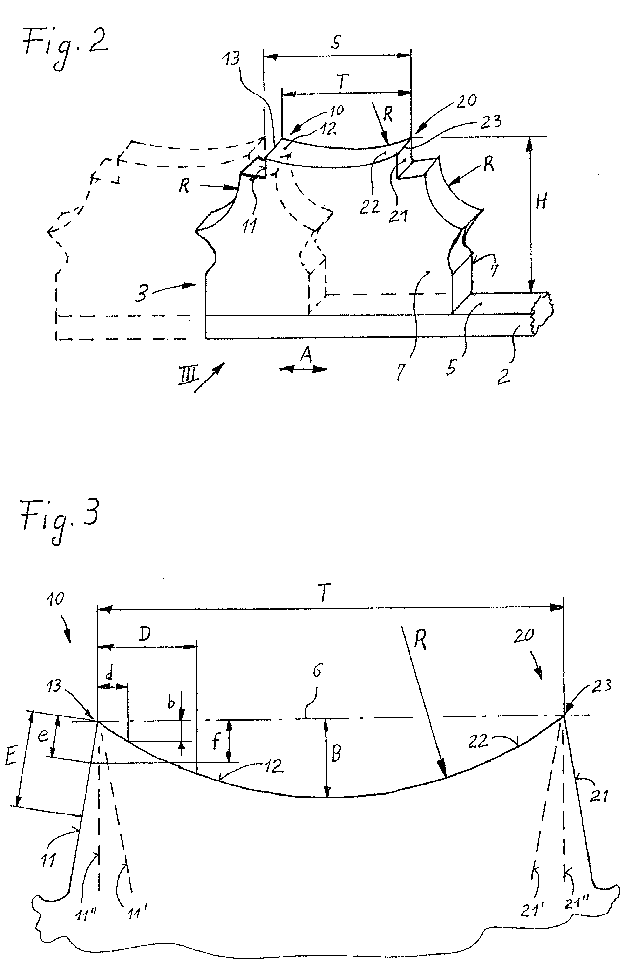

Im Folgenden wird die Ausgestaltung des ersten Zahnes 10 und des zweiten Zahnes 20 am Kopf 3 des Werkzeugs 1 mit Hilfe der Figuren 2 und 3 näher erläutert. Jeder der Zähne 10, 20 enthält eine erste Fläche 11, 21 zum Abschaben von Knochenmaterial, die quer zur Arbeitsrichtung A orientiert ist und als "Schabefläche" bezeichnet wird. Jeder der Zähne 10, 20 enthält eine zweite Fläche 12, 22 zum Andrücken von Knochenmaterial, die an die Schabefläche 11, 21 angrenzt und als "Andrückfläche" bezeichnet wird. Die Schabeflächen 11, 21 sind in Bezug auf die Arbeitsrichtung A stärker als die Andrückflächen 12, 22 geneigt. Die beiden Zähne 10, 20 sind mit ihrer Schabefläche 11, 21 und ihrer Andrückfläche 12, 22 entgegengesetzt orientiert, sodass bei einer Bewegung des Kopfes 3 in Arbeitsrichtung A jeweils die Schabefläche des in Arbeitsrichtung vorne liegenden Zahnes und die Andrückfläche des in Arbeitsrichtung dahinter liegenden Zahnes arbeiten. Wird beispielsweise der Kopf 3 entlang der Arbeitsrichtung A in den Figuren 2 und 3 von rechts nach links bewegt, so ist der Zahn 10 der in Arbeitsrichtung A vorne liegende Zahn und seine Schabefläche 11 arbeitet und schabt Knochenmaterial vom Knochen ab. Gleichzeitig ist der Zahn 20 der in Arbeitsrichtung A dahinter liegende Zahn, wobei dessen Andrückfläche 22 arbeitet und Knochenpartikel, die von der Schabefläche 11 abgeschabt worden sind, an den Grund der Nut andrückt, sodass dort das Knochenmaterial verdichtet wird. Bei der Rückwärtsbewegung des Kopfes 3 in den Figuren 2 und 3 von links nach rechts kehren sich die Verhältnisse um und der Zahn 20 ist der in Arbeitsrichtung A vorne liegende Zahn, dessen Schabefläche 21 arbeitet. Gleichzeitig ist der Zahn 10 der in Arbeitsrichtung A dahinterliegende Zahn, dessen Andrückfläche 12 arbeitet. Bei jeder Vorwärts- und Rückwärtsbewegung des Kopfes 3 wird somit von einer in Arbeitsrichtung A vorne liegenden Schabefläche Knochenmaterial abgeschabt, und von einer in Arbeitsrichtung A dahinterliegenden Andrückfläche wird abgeschabtes Knochenmaterial an den Knochen angedrückt. Hierdurch lässt sich eine sehr präzise Nut fräsen, wobei die Knochenoberfläche durch das von den Andrückflächen 12, 22 angedrückte Knochenmaterial verdichtet wird.

Jeder der Zähne 10, 20 enthält eine am Übergang von der Schabefläche 11 beziehungsweise 21 zur Andrückfläche 12 beziehungsweise 22 gebildete Kante 13 beziehungsweise 23, die als "Hauptschneide" bezeichnet wird. Für die weitere Beschreibung der Geometrie der Zähne 10 und 20 wird eine strichpunktiert dargestellte Referenzlinie 6 definiert, die eine Gerade ist, welche parallel zur Arbeitsrichtung A an die Hauptschneide 13 des ersten Zahnes 10 und die Hauptschneide 23 des zweiten Zahnes 20 angelegt wird. Die Hauptschneiden 13 und 23 liegen somit auf der gleichen Höhe H über der Führungsfläche 5. Außerdem wird eine Referenzebene definiert, welche parallel zur Arbeitsrichtung A und parallel zu der Zustellbewegung Z orientiert ist. Die Referenzebene liegt somit parallel zu der Zeichenebene in Figur 3 und ist zudem senkrecht zur Führungsfläche 5. Die Seitenflächen 7 des Kopfes 3 sind ebene Flächen, die parallel zur Referenzebene liegen. Der Abstand der Seitenflächen 7 zueinander kann 2 mm bis 4 mm betragen.

Die Andrückfläche 12 verändert in einem an die Hauptschneide 13 angrenzenden Abschnitt D ihren Abstand b zu der Referenzlinie 6 um 0,07 mm bis 0,17 mm, insbesondere um 0,09 mm bis 0,16 mm, wenn der Abstand b der Andrückfläche 12 zu der Referenzlinie 6 an zwei Stellen gemessen wird, die entlang der Referenzlinie 6 um ein Maß d von 0,2 mm voneinander beabstandet sind. Der Abschnitt D erstreckt sich von der Hauptschneide 13 bis zu einem Abstand von 1 mm entlang der Referenzlinie 6. Die Schabefläche 11 verändert in einem an die Hauptschneide 13 angrenzenden Abschnitt E ihren Abstand f zu der Referenzlinie 6 um 0,19 mm bis 0,2 mm, wenn der Abstand f der Schabefläche 11 zu der Referenzlinie 6 an zwei Stellen gemessen wird, die entlang der Schabefläche 11 um ein Maß e von 0,2 mm voneinander beabstandet sind. Der Abschnitt E erstreckt sich von der Hauptschneide 13 bis zu einem Abstand von 1 mm entlang der Schabefläche 11. Die Schabefläche 11 kann, wie in Figur 3 mit dem Bezugszeichen 11' gestrichelt angedeutet auch in die andere Richtung in Bezug auf die Referenzlinie 6 geneigt sein. Bevorzugt ist die Schabefläche 11 eine in Figur 3 mit dem Bezugszeichen 11" gestrichelt angedeutete, ebene Fläche, die senkrecht zur Arbeitsrichtung A orientiert ist, sodass der Abstand f sich um 0,2 mm verändert, wenn der Abstand f der Schabefläche 11 zu der Referenzlinie 6 an zwei Stellen gemessen wird, die entlang der Schabefläche 11 um das Maß e von 0,2 mm voneinander beabstandet sind. Die Schabefläche 11" weist die größtmögliche Neigung zur Arbeitsrichtung A auf. Die vorstehend für die Schabefläche 11 und die Andrückfläche 12 beschriebenen Abmessungen, insbesondere die Abstände b und f in Bezug auf die Referenzlinie 6, gelten genauso für die Schabefläche 21 und die Andrückfläche 22 des zweiten Zahnes 20. Die Zähne 10 und 20 sind spiegelsymmetrisch zu einer senkrecht zur Arbeitsrichtung liegenden Ebene.

Die Zahnkontur zwischen der Hauptschneide 13 des ersten Zahnes 10 und der Hauptschneide 23 des zweiten Zahnes 20 weist einen Abstand B zu der Referenzlinie 6, auf der höchstens 20 % des entlang der Referenzlinie 6 gemessenen Abstandes T von der Hauptschneide 13 des ersten Zahnes 10 zu der Hauptschneide 23 des zweiten Zahne 20 beträgt. Der Abstand T beträgt 4 mm bis 5 mm, sodass bei der Verwendung des Werkzeugs 1 in Stichsägen mit einem Hub zwischen 5 mm und 10 mm eine bestimmte Stelle des Knochens stets noch von beiden Hauptschneiden 13, 23 überstrichen wird. Der Hub der chirurgischen Stichsäge ist in Figur 2 mit dem Maß S zu einem gestrichelt dargestellten Kopf 3 angedeutet, welcher in Arbeitsrichtung A verschoben ist. Das Werkzeug hat eine Gesamtlänge von etwa 100 mm bis 150 mm, wobei der Kopf 3 eine Länge von 15 mm bis 20 mm und eine Breite von 2 mm bis 4 mm aufweist.

Die Andrückfläche 12 und die Andrückfläche 22 sind mit einem Krümmungsradius R konkav gewölbt. Die Andrückfläche 12 geht knickfrei in die Andrückfläche 22 über. Die Andrückfläche 12 und 22 wird durch die Oberfläche eines Kreiszylinders mit einem Durchmesser von 7 mm bis 11 mm gebildet. Der Krümmungsradius R beträgt dann 3,5 mm bis 5,5 mm. Die Mittellinie des Kreiszylinders liegt senkrecht zur Arbeitsrichtung A und gleichzeitig senkrecht zu der Referenzebene. Die Andrückflächen 12, 22 verlaufen senkrecht zur Referenzebene. Die Hauptschneiden 13 und 23 verlaufen geradlinig senkrecht zur Arbeitsrichtung A und senkrecht zur Referenzebene. Die Außenkontur des Kopfes 3 ist in einer Ansicht senkrecht auf die Referenzebene halbkreisförmig. Hierdurch ist der Kopf 3 optimal an die Verankerungsfinne eines Tibiaimplantants angepasst, welche an ihrem vorderen und hinteren Ende einen Radius aufweist, der dem Radius des die Hauptschneiden des Kopfes 3 umgebenden Halbkreises entsprechen kann.

Die zusätzlichen Zähne 30 können jeweils in gleicher Weise eine Schabefläche und eine Andrückfläche aufweisen und die gleiche Form wie die Zähne 10 und 20 haben. Sie üben ihre Schabe- und Andrückfunktion vor allem dann aus, wenn der Kopf 3 mit einer nicht senkrecht zur Führungsfläche 5 orientierten Zustellbewegung Z in den Knochen zugestellt wird. Die Referenzlinie in Bezug auf zwei hintereinanderliegende Zähne 30 wird dabei in der unverändert bleibenden Referenzebene an die Hauptschneiden dieser beiden Zähne 30 angelegt, sodass sie nicht mehr parallel zur Arbeitsrichtung A verläuft. Die Abmessungen R, T, B, b und f können dann analog in Bezug auf die anders liegende Referenzlinie bestimmt werden.

Ein Werkzeug, das für die Präparation einer Tibia-Nut zum Einsetzen des tibialen Anteils des Oxford-Kniesystems besonders geeignet ist, weist einen Abstand T von 5 mm, einen Radius R von 4 mm, eine Höhe H der beiden Hauptschneiden 13 und 23 von 12,8 mm auf. Der Abstand der ebenen Seitenflächen beträgt 2,8 mm für eine fit&fill-zementierte und 2,5 mm für eine pressfit-zementfreie Verankerung. Die ebenen Schabeflächen 11" und 21" sind hierbei senkrecht zur Referenzlinie 6.

### Bezugszeichenliste

- 1: Werkzeug
- 2: Körper
- 3: Kopf
- 4: Aufnahmebereich
- 5: Führungsfläche
- 6: Referenzlinie
- 7: Seitenflächen
- 8: Zentrierflächen
- 10: Erster Zahn
- 11: Schabefläche
- 12: Andrückfläche
- 13: Hauptschneide
- 20: Zweiter Zahn
- 21: Schabefläche
- 22: Andrückfläche
- 23: Hauptschneide
- 30: Weitere Zähne

- A: Arbeitsrichtung
- B: Abstand
- D: Abschnitt
- E: Abschnitt
- R: Krümmungsradius
- S: Hub
- T: Abstand
- Z: Zustellbewegung

- b: Abstand
- d: Maß
- e: Maß
- f: Abstand

## Patentansprüche

1. Werkzeug zum Einsetzen in eine chirurgische Stichsäge mit einem an eine Werkzeugaufnahme der chirurgischen Stichsäge angepassten Aufnahmebereich (4) und mit einem Kopf (3), der von der chirurgischen Stichsäge entlang einer Arbeitsrichtung (A) oszillierend bewegbar ist und mehrere Zähne (10, 20) aufweist, die in Arbeitsrichtung (A) hintereinander angeordnet sind,
wobei das Werkzeug (1) einen länglichen Körper (2) mit einer Längsrichtung enthält,
wobei an einem ersten Ende des Körpers (2) der Kopf (3) des Werkzeugs (1) angeordnet ist, und
wobei an einem zweiten Ende des Körpers (2) der Aufnahmebereich (4) angeordnet ist, über den das Werkzeug (1) von der chirurgischen Stichsäge in der parallel zur Längsrichtung des Körpers (2) orientierten Arbeitsrichtung (A) linear bewegbar ist, wobei der Kopf (3) des Werkzeugs (1) einen ersten (10) und einen zweiten (20) Zahn aufweist, wobei jeder der Zähne (10; 20) eine erste Fläche (11; 21) zum Abschaben von Knochenmaterial enthält, die quer zur Arbeitsrichtung (A) orientiert ist und eine Schabefläche bildet, wobei jeder der Zähne (10; 20) eine zweite Fläche (12; 22) zum Andrücken von Knochenmaterial enthält, die an die Schabefläche (11; 21) angrenzt und eine Andrückfläche bildet, wobei die Schabefläche (11; 21) in Bezug auf die Arbeitsrichtung (A) stärker als die Andrückfläche (12; 22) geneigt ist, und
wobei die beiden Zähne (10, 20) mit ihrer Schabefläche (11; 21) und ihrer Andrückfläche (12; 22) entgegengesetzt orientiert sind, sodass bei einer Bewegung des Kopfes in Arbeitsrichtung (A) jeweils die Schabefläche (11; 21) des in Arbeitsrichtung (A) vorne liegenden Zahnes (10; 20) und die Andrückfläche (22; 12) des in Arbeitsrichtung dahinter liegenden Zahnes (20; 10) arbeiten.

2. Werkzeug nach Anspruch 1, in welchem die Andrückfläche (12) des ersten Zahnes (10) und die Andrückfläche (22) des zweiten Zahnes (20) konkav gewölbt sind.

3. Werkzeug nach einem der vorstehenden Ansprüche, in welchem jeder der Zähne (10; 20) eine am Übergang von der Schabefläche (11; 21) zur Andrückfläche (12; 22) gebildete Kante (13; 23) enthält, die eine Hauptschneide bildet, wobei die Hauptschneide (13) des ersten Zahnes (10) parallel zu der Hauptschneide (23) des zweiten Zahnes (20) verläuft.

4. Werkzeug nach Anspruch 3, in welchem die Andrückfläche (12; 22) in einem an die Hauptschneide (13; 23) angrenzenden Abschnitt (D) ihren Abstand (b) zu einer Referenzlinie (6) höchstens um 0,2 mm verändert,
wenn der Abstand (b) der Andrückfläche (12; 22) zu der Referenzlinie (6) an zwei Stellen gemessen wird, die entlang der Referenzlinie (6) um 0,2 mm (d) voneinander beabstandet sind,
wobei die Referenzlinie (6) eine Gerade ist, die parallel zu der Arbeitsrichtung (A) an die Hauptschneide (13) des ersten Zahnes (10) und/oder die Hauptschneide (23) des zweiten Zahnes (20) angelegt wird.

5. Werkzeug nach nach Anspruch 4, in welchem die Schabefläche (11; 21) in einem an die Hauptschneide (13; 23) angrenzenden Abschnitt (E) ihren Abstand (f) zu der Referenzlinie (6) mindestens um 0,18 mm verändert,
wenn der Abstand (f) der Schabefläche (11; 21) zu der Referenzlinie (6) an zwei Stellen gemessen wird, die entlang der Schabefläche (11; 21) um 0,2 mm (e) voneinander beabstandet sind.

6. Werkzeug nach einem der ansprüche 4 und 5, in welchem die Zahnkontur zwischen der Hauptschneide (13) des ersten Zahnes (10) und der Hauptschneide (23) des zweiten Zahnes (20) einen Abstand (B) zu der Referenzlinie (6) aufweist, der höchstens 25 Prozent des entlang der Referenzlinie (6) gemessenen Abstandes (T) von der Hauptschneide (13) des ersten Zahnes (10) zu der Hauptschneide (23) des zweiten Zahnes (20) beträgt.

7. Werkzeug nach einem der vorstehenden Ansprüche, welches eine Führungsfläche (5) zum Begrenzen der Frästiefe des Werkzeugs (1) in dem Knochenmaterial aufweist,
wobei der Kopf (3) des Werkzeugs (1) über der Führungsfläche (5) vorsteht und die Führungsfläche (5) parallel zur Arbeitsrichtung (A) orientiert ist.

8. Werkzeug nach einem der Ansprüche 3 bis 6, oder nach Anspruch 7 wenn von einem der Ansprüche 3-6 abhängig, in welchem der Abstand (T) der Hauptschneide (13) des ersten Zahnes (10) zu der Hauptschneide (23) des zweiten Zahnes (20) - parallel zur Arbeitsrichtung (A) gemessen - höchstens so groß wie der Arbeitshub (S) der Säge ist, für die das Werkzeug bestimmt ist.

9. Werkzeug nach einem der Ansprüche 3 bis 6 oder 8, oder nach Anspruch 7 wenn von einem der Ansprüche 3-6 abhängig, in welchem die Breite einer der Hauptschneiden (13; 23) quer zur Arbeitsrichtung (A) der Breite der zu fräsenden Nut entspricht.

10. Verfahren zum Fräsen einer Nut in einen Knochen eines toten menschlichen oder tierischen Körpers oder in einen Knochen außerhalb des lebenden menschlichen oder tierischen Körpers
mit einem in eine chirurgische Stichsäge eingesetzten Werkzeug (1), welches einen Kopf (3) mit mehreren Zähnen (10, 20, 30) aufweist, der von der Stichsäge entlang einer Arbeitsrichtung (A) linear oszillierend vor und zurück bewegt wird,
in welchem bei der Vorwärtsbewegung des Kopfes (3) eine in Arbeitsrichtung (A) vorne liegende Schabefläche (11) eines ersten Zahnes (10) Knochenmaterial abschabt und eine in Arbeitsrichtung (A) hinter der Schabefläche (11) des ersten Zahnes (10) liegende Andrückfläche (22) eines zweiten Zahnes (20) Knochenmaterial an den Knochen andrückt, und
in welchem bei der Rückwärtsbewegung des Kopfes (3) eine in Arbeitsrichtung (A) vorne liegende Schabefläche (21) des zweiten Zahnes (20) Knochenmaterial abschabt und eine in Arbeitsrichtung (A) hinter der Schabefläche (21) des zweiten Zahnes (20) liegende Andrückfläche (12) des ersten Zahnes (10) Knochenmaterial an den Knochen andrückt.

11. Verfahren nach Anspruch 10, in welchem vor dem Fräsen der Nut in dem Knochen eine einen Schlitz enthaltende Frässchablone auf den Knochen aufgesetzt wird, und
in welchem anschließend der Kopf (3) des Werkzeugs (1) in den Schlitz der Frässchablone eingeführt wird, um eine Nut in das unter der Frässchablone liegende Knochenmaterial zu fräsen, die in ihrer Länge der Länge des Schlitzes in der Frässchablone und in ihrer Breite der Breite des Kopfes (3) des Werkzeugs (1) entspricht.

12. Verfahren nach Anspruch 10 oder 11, in welchem nach dem Fräsen der Nut in den Knochen ein Implantat in die Nut eingesetzt wird, wobei die Nutbreite in Abhängigkeit von der Breite des in die Nut einzusetzenden Teils des Implantats und in Abhängigkeit von der Art der Verankerung des Implantats gefräst wird.

## Claims

1. A tool for insertion into a surgical jigsaw, with a holder area (4) adapted to a tool holder of the surgical jigsaw and with a head (3), which is movable in oscillation by the surgical jigsaw along a work direction (A) and has a plurality of teeth (10, 20) which are arranged behind one another in the work direction (A),
wherein the tool (1) contains an elongated body (2) with a longitudinal direction,
wherein at a first end of the body (2) the head (3) of the tool (1) is arranged, and
wherein at a second end of the body (2) the holder area (4) is arranged via which holder area (4) the tool (1) is movable by the surgical jigsaw in the work direction (A) oriented parallel to the longitudinal direction of the body (2),
wherein the head (3) of the tool (1) has a first (10) and a second (20) tooth,
wherein each of the teeth (10; 20) contains a first surface (11; 21) for shaving off bone material, which surface is oriented transversely to the work direction (A) and forms a shaving surface,
wherein each of the teeth (10; 20) has a second surface (12; 22) for pressing on bone material, which surface adjoins the shaving surface (11; 21) and forms a pressing surface,
wherein the shaving surface (11; 21), in relation to the work direction (A), is more inclined than the pressing surface (12; 22), and
wherein the two teeth (10, 20) are oriented with their shaving surface (11; 21) and their pressing surface (12; 22) opposite each other, such that, during a movement of the head in work direction (A), at any one time the shaving surface (11; 21) of the tooth (10; 20) lying ahead in work direction (A) and the pressing surface (22; 12) of the tooth (20; 10) lying therebehind in work direction are in operation.

2. The tool according to one of the preceding claims, in which the pressing surface (12) of the first tooth (10) and the pressing surface (22) of the second tooth (20) are curved in a concave manner.

3. The tool according to one of the preceding claims, in which each of the teeth (10; 20) contains an edge (13; 23) formed at the transition from the shaving surface (11; 21) to the pressing surface (12; 22), which forms a main cutting edge,
wherein the main cutting edge (13) of the first tooth (10) runs parallel to the main cutting edge (23) of the second tooth (20).

4. The tool according to claim 3, in which the pressing surface (12; 22) in a section (D) adjoining the main cutting edge (13; 23) changes its distance (b) with respect to a reference line (6) at most by 0.2 mm,
when the distance (b) of the pressing surface (12; 22) to the reference line (6) is measured at two locations which are spaced apart from one another by 0.2 mm (d) along the reference line (6),
wherein the reference line (6) is a straight line which is applied parallel to the work direction (A) onto the main cutting edge (13) of the first tooth (10) and/or the main cutting edge (23) of the second tooth (20).

5. The tool according to claim 4, in which the shaving surface (11; 21) in a section (E) adjoining the main cutting edge (13; 23) changes its distance (f) with respect to the reference line (6) at least by 0.18 mm,
when the distance (f) of the shaving surface (11; 21) to the reference line is measured at two locations which are spaced apart from one another by 0.2 mm (e) along the shaving surface (11; 21).

6. The tool according to one of the claims 4 and 5, in which the tooth contour between the main cutting edge (13) of the first tooth (10) and the main cutting edge (23) of the second tooth (20) has a distance (B) with respect to the reference line (6) which is at most 25 percent of the distance (T), measured along the reference line (6), from the main cutting edge (13) of the first tooth (10) to the main cutting edge (23) of the second tooth (20).

7. The tool according to one of the preceding claims, which has a guide surface (5) for delimiting the milling depth of the tool (1) in the bone material,
wherein the head (3) of the tool (1) projects over the guide surface (5) and the guide surface (5) is oriented parallel to the work direction (A).

8. The tool according to one of claims 3 to 6, or according to claim 7 if depending from one of claims 3 to 6, in which the distance (T) of the main cutting edge (13) of the first tooth (10) to the main cutting edge (23) of the second tooth (20) - measured parallel to the work direction (A) - is at most as great as the working stroke (S) of the saw, for which the tool is intended.

9. The tool according to one of claims 3 to 6, or according to claim 7 if depending from one of claims 3 to 6, in which the width of one of the main cutting edges (13; 23) transversely to the work direction (A) corresponds to the width of the groove which is to be milled.

10. A method for milling a groove in a bone of a dead human or animal body or in bone outside the living human or animal body with a tool (1) inserted into a surgical jigsaw, which tool has a head (3) with a plurality of teeth (10, 20, 30), which head is moved in oscillation forward and backward by the jigsaw along a work direction (A),
in which method during the forward movement of the head (3) a shaving surface (11) of a first tooth (10), lying ahead in work direction (A), shaves off bone material and a pressing surface (22) of a second tooth (20), lying behind the shaving surface (11) of the first tooth (10) in work direction (A), presses bone material onto the bone, and
in which during the backward movement of the head (3) a shaving surface (21) of the second tooth (20), lying ahead in work direction (A), shaves off bone material and a pressing surface (12) of the first tooth (10), lying behind the shaving surface (21) of the second tooth (20) in work direction (A), presses bone material onto the bone.

11. The method according to claim 10, in which before the milling of the groove into the bone a milling template containing a slit is placed onto the bone, and in which subsequently the head (3) of the tool (1) is introduced into the slit of the milling template in order to mill a groove into the bone material lying beneath the milling template, which groove corresponds in its length to the length of the slit in the milling template and corresponds in its width to the width of the head (3) of the tool (1).

12. The method according to claim 10 or 11, in which after the milling of the groove into the bone, an implant is inserted into the groove, wherein the groove width is milled as a function of the width of the part of the implant which is to be inserted into the groove and as a function of the type of anchoring of the implant.

## Revendications

1. Outil destiné à être inséré dans une scie sauteuse chirurgicale avec une partie d'insertion (4) adaptée à la réception d'un outil de la scie sauteuse chirurgicale et une tête (3) qui peut se déplacer en oscillant le long d'un sens de travail (A) à partir de la scie sauteuse chirurgicale et présente plusieurs dents (10, 20) qui sont disposées les unes derrière les autres dans le sens du travail (A),
où l'outil (1) contient un corps (2) allongé avec une direction longitudinale,
où la tête (3) de l'outil (1) est disposée au niveau d'une première extrémité du corps (2), et
où la partie d'insertion (4) est disposée au niveau d'une deuxième extrémité du corps (2), par-dessus laquelle l'outil (1) de la scie sauteuse chirurgicale peut se déplacer linéairement dans le sens du travail (A) orienté parallèlement par rapport à la direction longitudinale du corps (2),
où la tête (3) de l'outil (1) présente une première (10) et une deuxième (20) dent,
où chacune des dents (10 ; 20) contient une première surface (11 ; 21) pour racler du matériau osseux, qui est orientée transversalement par rapport au sens du travail (A) et forme une surface de raclage,
où chacune des dents (10 ; 20) contient une deuxième surface (12 ; 22) pour une application par pression de matériau osseux, qui est limitrophe de la surface de raclage (11 ; 21) et forme une surface d'application par pression, où la surface de raclage (11 ; 21) est plus inclinée par rapport au sens du travail (A) que la surface d'application par pression (12 ; 22), et
où les deux dents (10, 20) sont orientées dans des sens opposés avec leur surface de raclage (11 ; 21) et leur surface d'application par pression (12 ; 22) de sorte que, lors d'un mouvement de la tête dans le sens du travail (A) respectivement, la surface de raclage (11 ; 21) de la dent (10 ; 20) située vers l'avant dans le sens du travail et la surface d'application par pression (22 ; 12) de la dent (20 ; 10) située derrière dans le sens du travail sont en fonctionnement.

2. Outil selon la revendication 1, dans lequel la surface d'application par pression (12) de la première dent (10) et la surface d'application par pression (22) de la deuxième dent (20) sont cambrées de manière concave.

3. Outil selon l'une des revendications précédentes, dans lequel chacune des dents (10 ; 20) contient une arête (13 ; 23) formée au niveau de la transition de la surface de raclage (11 ; 21) vers la surface d'application par pression (12 ; 22), qui forme un tranchant principal,
où le tranchant principal (13) de la première dent (10) s'étend parallèlement par rapport au tranchant principal (23) de la deuxième dent (20).

4. Outil selon la revendication 3, dans lequel la surface d'application par pression (12 ; 22) modifie d'au maximum 0,2 mm sa distance (b) par rapport à une ligne de référence (6) dans une section (D) limitrophe du tranchant principal (13 ; 23),
lorsque la distance (b) de la surface d'application par pression (12 ; 22) jusqu'à la ligne de référence (6) est mesurée au niveau de deux endroits qui sont distants l'un de l'autre de 0,2 mm (d) le long de la ligne de référence (6), où la ligne de référence (6) est une ligne droite qui est appliqué parallèlement au sens du travail (A) au niveau du tranchant principal (13) de la première dent (10) et/ou le tranchant principal (23) de la deuxième dent (20).

5. Outil selon la revendication 4, dans lequel la surface de raclage (11 ; 21) modifie d'au moins 0,18 mm sa distance (f) par rapport à la ligne de référence (6) dans une section (E) limitrophe du tranchant principal (13 ; 23), lorsque la distance (f) de la surface de raclage (11 ; 21) jusqu'à la ligne de référence (6) est mesurée au niveau de deux endroits qui sont distants l'un de l'autre de 0,2 mm (e) le long de la surface de raclage (11 ; 21).

6. Outil selon l'une des revendications 4 et 5, dans lequel le contour de dent entre le tranchant principal (13) de la première dent (10) et le tranchant principal (23) de la deuxième dent (20) présente une distance (B) par rapport à la ligne de référence (6) qui est égal au maximum à 25 pourcent de la distance (T) le long de la ligne de référence (6) mesurée du tranchant principal (13) de la première dent (10) jusqu'au tranchant principal (23) de la deuxième dent (20).

7. Outil selon l'une des revendications précédentes, lequel présente une surface de guidage (5) pour la délimitation de la profondeur de fraisage de l'outil (1) dans le matériau osseux,
où la tête (3) de l'outil (1) dépasse par-dessus la surface de guidage (5) et la surface de guidage (5) est orientée parallèlement par rapport au sens du travail (A).

8. Outil selon l'une des revendications 3 à 6, ou selon la revendication 7 lorsqu'elle est dépendante de l'une des revendications 3 à 6, dans lequel la distance (T) du tranchant principal (13) de la première dent (10) jusqu'au tranchant principal (23) de la deuxième dent (20), mesurée parallèlement par rapport au sens du travail (A), est au maximum aussi grande que la course de travail (S) de la scie pour laquelle l'outil est prévu.

9. Outil selon l'une des revendications 3 à 6 ou 8, ou selon la revendication 7 lorsqu'elle est dépendante de l'une des revendications 3 à 6, dans lequel la largeur de l'un des tranchants principaux (13 ; 23) transversalement par rapport au sens du travail (A) correspond à la largeur de la rainure à fraiser.

10. Procédé de fraisage d'une rainure dans un os d'un corps mort humain ou animal ou dans un os en dehors du corps vivant humain ou animal avec un outil (1), inséré dans une scie sauteuse chirurgicale, lequel présente une tête (3) avec plusieurs dents (10, 20, 30), qui est déplacé en oscillant linéairement vers l'avant et l'arrière par la scie sauteuse le long d'un sens de travail (A),
dans lequel, lors du mouvement de la tête (3) en avant, une surface de raclage (11) d'une première dent (10) située devant dans le sens du travail (A) racle du matériau osseux et une surface d'application par pression (22) d'une deuxième dent (20) située derrière la surface de raclage (11) de la première dent (10) dans le sens du travail (A) applique par pression le matériau osseux contre l'os, et
dans lequel, lors du mouvement de la tête (3) en arrière, une surface de raclage (21) de la deuxième dent (20) située à l'avant dans le sens du travail (A) racle du matériau osseux et une surface d'application par pression (12) de la première dent (10) située derrière la surface de raclage (21) de la deuxième dent (20) dans le sens du travail (A) applique par pression le matériau osseux sur l'os.

11. Procédé selon la revendication 10, dans lequel, avant le fraisage de la rainure dans l'os, un gabarit de fraisage contenant une fente est disposé sur l'os, et
dans lequel, ensuite, la tête (3) de l'outil (1) est insérée dans la fente du gabarit de fraisage afin de fraiser une rainure dans le matériau osseux se situant en dessous du gabarit de fraisage, qui correspond dans sa longueur à la longueur de la fente dans le gabarit de fraisage et dans sa largeur à la largeur de la tête (3) de l'outil (1).

12. Procédé selon la revendication 10 ou 11, dans lequel, après le fraisage de la rainure dans l'os, un implant est inséré dans la rainure, où la largeur de rainure est fraisée en fonction de la largeur de la partie de l'implant à insérer dans la rainure et en fonction du type d'ancrage de l'implant.
